# EUROPEAN PATENT SPECIFICATION

(11) **EP 1 353 968 B1**
(45) Date of publication and mention of the grant of the patent: **15.11.2006**
(21) Application number: 01273000.8
(22) Date of filing: 27.12.2001
(51) Int. Cl.: C08G 18/10, C08G 18/32, A61L 27/34, A61L 15/42, A61K 47/30

(54) **LINEAR BLOCK COPOLYMER**
LINEARES BLOCKCOPOLYMER
COPOLYMERE SEQUENCE LINEAIRE

(30) Priority: 29.12.2000 SE 0004924; 02.03.2001 SE 0100735
(43) Date of publication of application: 22.10.2003
(73) Proprietor: Artimplant AB, 421 32 Västra Frölunda (SE)
(72) Inventor: AURELL, Carl-Johan, S-431 40 Mölndal (SE); FLODIN, Per, S-436 55 Hovas (SE)
(74) Representative: Romare, Laila Anette
(86) International application number: PCT/SE2001/002902
(87) International publication number: WO 2002/053617

(56) References cited:
- WO-A1-00/45869
- WO-A1-97/22643
- US-A- 4 265 973
- US-A- 4 743 632
- US-A- 4 797 430
- US-A- 5 045 622
- DATABASE WPI Week 199933, Derwent Publications Ltd., London, GB; AN 1999-389428, XP002950633 'Manufacture of polyurethane urea resin for coating agents, coating materials, adhesives, etc - involves reacting isocyanate terminated prepolymer with compound containing primary or secondary amino groups and hydroxyl group' & JP 11 152 322 A (TOYOBO KK) 08 June 1999
- DATABASE CA [Online] (COLUMBUS, OHIO, US) 'Preparation of hydroxy-functional thermoplastic polyurethane-polyureas', XP002950632 Retrieved from STN Database accession no. 131:45257 & JP 11 152 322 A 08 June 1999

## Description

### FIELD OF THE INVENTION

The present invention relates to a new linear block polymer, method for preparation and method for fractional precipitation of said linear block polymer, use of said linear block polymer, implants for humans and animals, which implants comprises said linear block polymer, material for promoting healing of wounds of humans and animals, which materials comprises said linear block polymer and also pharmaceutical compositions, microencapsules, suspensions, emulsions, which all comprise said linear block polymer.

### PRIOR ART

At injuries or illness in humans or animals, damaged organs or damaged tissues must sometimes be replaced temporarily or permanently by some kind of implant. For such an implant to have an acceptable function, the implant must, firstly, have properties, e.g. strength, which properties make the implant able to replace the functions of the damaged organ or tissue, and, secondly, it must be bio-compatible. Different materials, such as pure titanium and some kinds of plastic materials have been shown to have acceptable function and are already used to a great extent. Often it is desired that an implant promotes growth of the damaged tissue at the same time as the implant in many cases should be biologically degradable.

In SE, C2, 505703 there is described a linear block polymer having a molecular weight of 10⁴ Dalton, preferably 10⁵ Dalton, comprising urea and urethane groups and ester groups at such distance from each other that after their hydrolysis the resulting fragments are that small that they can be secreted from a human body. Said linear block polymer comprising urea and urethane groups is suitable as material in implants for humans and animals.

### DESCRIPTION OF THE INVENTION

The present invention relates to a linear block polymer having a molecular weight of at least 10⁴ Dalton, which linear block polymer is comprised of internally linearly connected sequences, which sequences may be described according to formula (1) 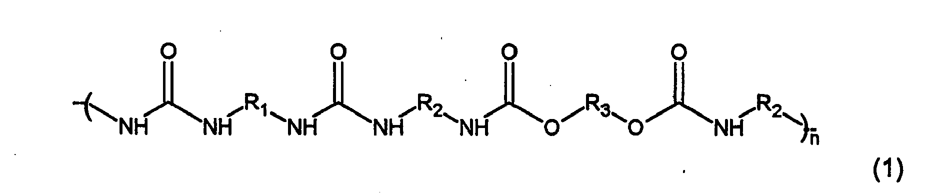 wherein
1 to 100 % of R₁, which 1 to 100 % of R₁ may be the same or different, comprise a segment which is described according to formula (1a) 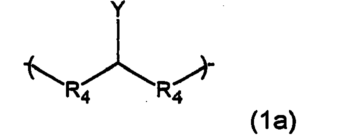 wherein Y, which is a substituent, is R₅-Z, wherein
R₅ is (C₀-C₆) alkyl, and
each Z, which Z may be the same or different, is a protected or unprotected functional group such as hydroxyl, amino, carboxyl, thiol, cyano, or nitro group, and
each R₄, which R₄ may be the same or different, is (C₁-C₆) alkyl; and,
when not 100 % of R₁ comprises a segment according to formula (1 a) as above, may each other R₁, which each other R₁, the same or different, do not comprise a segment according to formula (1 a), be derived from aliphatic or aromatic diamines;
each R₂, which R₂ may be same or different, may be derived from a diisocyanate and comprises no, one or more ester groups;
each R₃, which R₃ may be same or different, comprises no, one or more ester groups and may be derived from one or more of polyesterdiol, polyetherdiol or monodiol, or
each R₃, which R₃ may be the same or different, may be described according to formula (1 b) 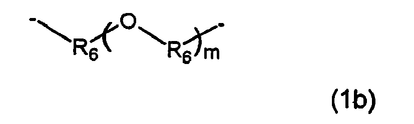 wherein each R₆ is (C₂₋C₄) alkyl, and
m is 1 to 6; and
wherein each R₂ and/or R₃ comprise one or more ester groups; and
n is in the interval from 10 to 1000 which gives said linear block polymer a molecular weight of at least 10⁴ Dalton.

Said linear block polymer is of the polyurethaneurea kind as the polymer chain contains' urethane as well as urea groups. Both urethane and urea groups in the block polymer form intermolecularly hydrogen bonds, which gives the cohesive forces which are needed to hold the molecules together to a material. From urea groups there are obtained especially strong intermolecular forces, and especially when several urea groups are given the possibility to co-operate. The blocks in a block polymer which contain urea groups are often called "hard" since they are responsible for the cohesion of the material, wherein the cohesion is a function of the number of and the length of the blocks which contain urea groups. In said linear block polymer according to formula (1) the "hard" block is that block which is comprised of R₁ and the adjacent urea groups. Correspondingly the block in a block polymer which gives the material stretchability and elasticity are often called "soft". In said linear block polymer according to formula (1) the "soft" block is that block which is comprised of R₃, and also the adjacent urethane groups may be comprised in the soft block.

When R₁ comprises a segment which may be described according to formula (1 a), R₁ also comprises Z, a functional group, which group is suitable for a covalent chemical bonding, wherein said bonding may be reversible or irreversible. During preparation of said linear block polymer Z may be protected or unprotected, Z shall be protected if Z may interfere with the preparation. R₅ is (C₀-C₆) alkyl which means that R₅ is missing, alternatively said to be a bond, or is an alkyl having up to 6 carbon atoms. Further, that 1 to 100 % of R₁ comprises a segment which may be described according to formula (1a) means that the amount of Z in said linear block polymer may be chosen after desire. For example, if a maximum amount of Z in said linear block polymer according to the present invention is desired, then 100 % of R₁ shall comprise a segment which may be described according to formula (1a). When the percentage, i.e. the amount, of R₁ which comprises a segment which may be described according to formula (1a) decreases will also the amount Z in said linear block polymer according to the present invention decrease. Examples of different percentages of R₁, which R₁ comprises a segment which may be described according to (1a), in said linear block polymer according to the present invention is 1, 2, 5, 10, 20, 40, 80 and 100 %.

When not a 100 % of R₁ comprises a segment according to formula (1a), the other R₁, the same or different, which do not comprise a segment according to formula (1a), may be derived from aliphatic or aromatic diamines. Examples of these diamines are primary diamines, e.g. ethylene diamine, 1,3-diaminopropane, 1,3-propanediol-bis-p-aminobenzoate or ethyleneglycol bisglycinester diamine.

Principally, R₃ may be derived from an optional diol provided that the diol does not contain other groups which are reacting with isocyanate than hydroxyl groups.

Said linear block polymer is suitable as material in implants for humans or animals and comprises secondary functional groups for covalently chemical bonding, for example secondary hydroxyl, amino, carboxyl, and/or thienyl groups, wherein biologically active substances may be covalently bonded, reversibly or irreversibly, to said linear block polymer. Further, said linear block polymer comprises ester groups at such a distance from each other that after hydrolysis of said ester groups, the resulting fragments is less than 2000 Dalton, wherein the fragments may be secreted from a human or animal body. Preferably, the resulting fragments are less than 1000 Dalton.

According to the present invention, a linear block polymer is disclosed comprising urea and urethane groups which is suitable for implants, wherein said linear block polymer is biologically degradable in a human or animal body and has ability that through a coupled substance show biological activity. The biological activity may be shown when the substance is coupled to said linear block polymer and/or when a hydrolysable covalent bonding which couples the substance to said linear block polymer is hydrolysed in a human or animal body. Further, the biological activity may be shown when said linear block polymer is hydrolysed and degraded in a human or animal body.

Further said linear block polymer according to formula (1) has been shown to have a very useful feature, the feature that it is possible to precipitate said linear block polymer and to then redissolve the very same. This feature is strongly connected with that said linear block polymer comprises interfering, among other functional, groups in the "hard" block. To be able to precipitate a linear block polymer in ethanol or water is a very useful feature when the final block polymer shall be chemically modified, since many reagents and substrates are soluble in water and ethanol. Thereby it is possible to purify a chemically modified block polymer by precipitation. The linear block polymer may be dissolved in a solvent for example dimethylformamide, dimethylsulfoxide (DMSO), N,N-dimethylacetamide (DMAC) or N-methylpyrrolidone (NMP), and has the feature to be able to be precipitated in ethanol or water. Further, the molecular weight distribution of said linear block polymer may be changed by precipitating and solving said linear block polymer. Undesired molecular weights may for example be sorted and separated out by a fractional precipitation.

The molecular weight of the linear block polymer is essential for the mechanical features of the linear block polymer.

A further embodiment according to the present invention relates to a linear block polymer, wherein R₅ is missing, or alternatively may be said to be a bond, i.e., R₅ is C₀-alkyl.

Still a further embodiment according to the present invention relates to a linear block polymer, wherein 10 to 100 % of R₁, which 10 to 100 % of R₁ may be the same or different, comprises a segment which may be described according to formula (1a) above.

Further, the present invention relates to an embodiment wherein a linear block polymer, wherein 100 % of R₁, which 100 % of R₁ may be the same or different, comprises a segment which may be described according to formula (1a) as above. It means that each R₁, which R₁ may be the same or different, here comprises a segment which may be described according to formula (1a).

R₁ comprises a segment, which may be described according to formula (1a), and thereby also Z, a functional group, which group is suitable for covalent chemical bonding, wherein said bonding may be reversible or irreversible. During preparation of said linear block polymer Z may be protected or unprotected, Z shall be protected if Z can interfere with said preparation.

Further, said linear block polymer according to formula (1) has shown to have a very useful feature in that it is possible to precipitate and then redissolve said linear block polymer. Said feature may be mostly pronounced when all R₁ comprises a segment which may be described according to formula (1a).

A further embodiment according to the present invention relates to a linear block polymer according to formula (1), wherein each R₂, which R₂ may be the same or different, may be derived from diphenylmethanediisocyanat (MDI), hexamethylenediisocyanat (HDI), 1-isocyanato-4-[(4-isocyanatocyclohexyl)methyl]cyclohexane (H₁₂MDI) or ethyl-2,6-diisocyanatohexanoate (LDI).

Still a further embodiment according to the present invention relates to a linear block polymer according to formula (1) wherein R₃ may be derived from one or more of polytetramethyleneoxidediol, polyethyleneoxidediol, polycaprolactonediol, polyethyleneglycoladipatediol, polyldiethyleneglycoladipatdiol, glycerinemonoallylether, trimethylolpropanemonallylether, glycerinemonoglycidylether, dimethylolpropionacidmethylester, dimethylolpropionacidbrombutylester, esters of monocarboxymethylethers of glycerine, or trimethylpropane.

Even a further embodiment according to the present invention relates to a linear block polymer according to formula (1), wherein
each R₂ comprises one or more ester groups and/or
each R₃ comprises on or more ester groups.

A further embodiment according to the present invention relates to a linear block polymer, wherein each Z, which Z may be the same or different, is a hydroxyl, amino, carboxyl, thiol, cyano, or nitro group.

Still a further embodiment according to the present invention relates to a linear block polymer, wherein said linear block polymer has a molecular weight of at least 5*10⁴ Dalton.

Even a further embodiment according to the present invention relates to a linear block polymer, wherein said linear block polymer comprises a biologically active substance, wherein said biologically active substance is covalently bonded to Z. The biological activity may be exhibited when the substance is reversibly or irreversibly coupled to said linear block polymer and/or when a hydrolysable covalent bonding which couples said substance to said linear block polymer is hydrolysed in a human or animal body. Further, the biological activity may be exhibited when said linear block polymer is hydrolysed and degraded in a human or animal body. Thus, by said linear block polymer it is possible to obtain a local application of said substance to a specific area, a prolonged application of the very same. Further, the biological activity may comprise growth promoting, anti-microbial or hormonal activity.

A further embodiment according to the present invention relates to a linear block polymer, wherein said biologically active substance for example may be biologically active peptides e.g. growth factors or GHK, i.e. glycylhistidyllysin, penicillins e.g. benzylpenicillin, fucidin acid or tetracyclins.

The present invention also relates to a method for preparation of a linear block polymer according to formula (1), wherein said method comprises chain-extension, wherein about n mol of a diamine according to formula (2) 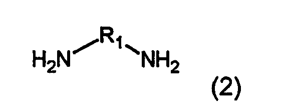 is reacted with about n mol of a urethanediisocyanate according to formula (3), 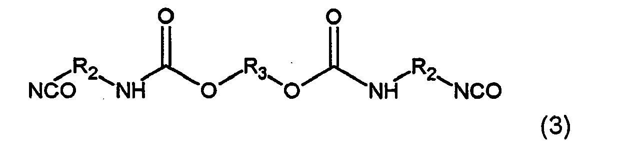 wherein R₁, R₂ and R₃ are defined as in formula (1) as above, provided that if R₁ comprises R₅-Z which may interfere with the preparation then R₅-Z is protected.

A molar ratio of -NH₂/-NCO from 0.95 to 1.05 in the above chain extension is used for achieving as high values of molecular weights of said linear block polymer according to formula (1). Further in the above chain extension, if in the R₁ which comprises a functional group, e.g. for covalent chemical bonding, i.e. Z, for example a protected or unprotected functional group such as hydroxyl, amino, carboxyl, thiol, cyano, or nitro group, said Z may interfere with said method Z shall be protected. When the functional group, Z, is protected, it may be protected with tert-butyloxycarbonyl-, fluorenylmethyloxycarbonyl-, benzyloxycarbonyl-, tert-butyl- protecting group or the like. Further, a carboxyl group may, for example, be protected by performing the preparation in an alkali environment.

Said urethanediisocyanate according to formula (3) may be prepared by reacting diol with a diisocyanat, according to

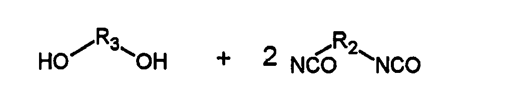

Further, the present invention relates to a method for preparing a linear block polymer according to formula (1), wherein said method comprises precipitation with alcohol, such as ethanol or isopropanol, or precipitation with water of said linear block polymer in solution.

To be able to precipitate a linear block polymer in ethanol or water is a very useful feature as described before.

Still a further embodiment relates to a method according to the present invention for preparing a linear block polymer according to formula (1), wherein said method further comprises covalent bonding of a biologically active substance to said linear block polymer, for example through primary or secondary functional groups for covalent bonding.

The covalent bonding and said biologically active substance may both be chosen in such way that biological activity may be exhibited when the substance is reversibly or irreversibly coupled to said linear block polymer and/or when a hydrolysable covalent bonding which couples the substance to said linear block polymer is hydrolysed in a human or animal body. Further, the biological activity may comprise growth promoting, anti-microbial or hormonal activity, and said biologically active substance may for example be biologically active peptides, e.g. growth factors or GHK, i.e. glycylhistidyllysin, penicillins e.g. benzylpenicillin, or fucidin acid, tetracyclins.

Still a further embodiment relates to a method according to the present invention for preparation of said linear block polymer according to formula (1), wherein said covalent bonding comprises bonding to functional groups for covalent bonding at said linear block polymer.

Still an embodiment relates to a method according to the present invention for fractional precipitation of a linear block polymer according to formula (1), wherein the method comprises precipitation and solving of said linear block polymer as described above. Thus, the molecular weight distribution of said linear block polymer may be changed. Undesired molecular weights may, for example, be sorted and separated out by a so called fractional precipitation.

Further the present invention also relates to use of a linear block polymer, for example in the form of fibres, film, moulded bodies, implants, pipes or an open porous polymer material, as for example materials in implants for humans or animals, for example as implants for tendons, ligaments, blood vessels, discs or menisci, at pharmaceutical compositions, at microencapsulation, in suspensions, in emulsions, in porous polymer materials or the like, or as filling material for example in bone such as discs, synthetic bone replacement, for example in the form of granules for replacement of bone tissue, menisci or the like, or as pipes for, for example, replacement of blood vessels, guidance for promotion/regeneration of tendons and/or nerves, or other biological tissue, or in connection to treatment of wounds as carrier of dressings for wound healing, growth factors or the like, artificial skin, or as matrix/scaffold for, for example: stem cells, fibroblasts, osteoblasts, osteocytes, chondroblasts, chondrocytes among other, as well as autogenous, allogenous as xenogenous, or as matrix/scaffold for promotion/regeneration of tissue, for example, tendons and/or nerves, or other biological tissue.

Ideally, a matrix/scaffold for tissue proliferation (or growth/regeneration should have the following characteristics; (i) three-dimensional and highly porous with an interconnected pore network for cell growth and flow transport of nutrients and metabolic waste; (ii) biocompatible and bioresorbable with a controllable degradation and resorption rate to match cell/tissue ingrowth in vitro and/or in vivo; (iii) suitable surface chemistry for cell attachment, proliferation and differentiation and (iv) mechanical properties to match those of the tissues at the site of implantation.

Porous polymer materials are for example described in Swedish Patent Application SE, A, 0004856-1, which is hereby incorporated as a whole. SE, A, 0004856-1 describes among other a method for preparation of an open porous polymer material.

Further embodiment relates to use of said linear block polymer according to the present invention as a material for promoting the healing of wounds in humans or animals.

The present invention also relates to implants for humans or animals, which implants comprises a linear block polymer according to formula (1).

Further, the present inventions relates to a material for filling in bones, promotion of wound healing, replacement for blood vessels, guidance for growth/regeneration of tendons and/or nerves, or other biological tissue in humans or animals, which material comprises a linear block polymer according to formula (1).

The present inventions also relates to pharmaceutical compositions, microencapsules, suspensions, emulsions, matrix/scaffolds for growth/regeneration of tissue, for example, tendons and/or nerves, or other biological tissue, which comprises a linear block polymer according to formula (1).

Still a further embodiment according to the present invention relates to a porous polymer material, which polymer material comprises a linear block polymer according to formula (1). Said porous polymer material may, for example, be an open porous polymer material according to said Swedish Patent Application SE, A, 0004856-1, which polymer material has internally connected pores, i.e. a continuos structure of pores. When using said open porous polymer material according to the invention, wherein the said Z, i.e. the protected or unprotected functional group, is localised in a polymer segment which must be considered as hard and rigid, it has likewise shown that said Z in the polymer material is well exposed to reactants in solutions in pores of the polymer material, wherein Z is available for chemical reactions. In an example wherein Z is a hydroxyl group having a coupled benzyl penicillin in said open porous polymer material, a sulphur analysis indicates that 5 % of the hydroxyl groups in the open porous polymer material have bonded benzyl penicillin. Further, by coupling of molecules which are sterically less hindered, e.g. glycine, an amino acid analysis shows that an increase in the bonding to the hydroxyl groups is obtained. It has also been shown that when the coupling, the bonding, is achieved in a solution of DMF a 5-10 times increase in the coupling or bonding to the hydroxyl groups is obtained.

### EXAMPLES

Glucose-monohydrate and benzylpenicillin were obtained from Applichem , Darmstadt, Germany and were of bio-grade quality. EDC-HCl was obtained from Senn Chemicals, Dielsdorf, Switzerland. DMF (anhydroscan) was obtained from LAB SCAN, Dublin, Ireland and was of HPLC quality. MDI was obtained from Bayer AG, Leverkusen, Germany and the polycaprolacton diol was obtained from Solvay Interox LTD, Warrington, England. All other chemicals were obtained from Sigma-Aldrich-Fluka and were of analytical reagent quality.

NMR spectra were recorded on a Varian VH 300 MHz instrument. The content of sulphur was analysed on a LECO SC-432 Sulphur Analyzer at Mikro Kemi AB in Uppsala, Sweden. Amino acid analyses were performed by Aminosyraanalyscentralen at the Biomedical Center at Uppsala university, Sweden.

### Example 1

### Preparation of a polymer "POL 4040" by chain extension of a pre-polymer (PRE M0006) with 1,3-diamino-2-hydroxypropane.

A pre-polymer (PRE M0006) was prepared by reacting diphenylmethanediisocyanat (MDI) with polycaprolactonediol (PLC 530) in a molar ratio 2:1, i.e. [NCO:OH = 2:1].

Before the preparation of the polymer the glass equipment was dried in 77°C at about 2 h.

| Mol ratio | n (mmol) | Mw (g/mol) | m (g) | Substance |
|---|---|---|---|---|
| 1 | 30.6 | 994.78 | 30.46 | PRE M0006 |
| 0.98 | 30.0 | 90.12 | 2.704 | 1,3-diamino-2-OH-propane |
| 0.02 | 0.612 | 129.25 | 0.079 | Dibutylamin |
| | | | 181.228 | DMF |

The desired end concentration of polymer "POL 4040" in DMF was decided to 15.5 % by weight.

The pre-polymer (PRE M0006) was weighed in a 1 I flask.

30.46 g of the pre-polymer was dissolved in 133.6 ml dimethylformamide (DMF), i.e. in 70 % of the total amount of DMF. The mixture was stirred under a nitrogen environment until a clear solution was obtained, which took about 20 minutes. 2.7 g 1,3-diamino-2-hydroxypropane and 0.079 g dibutylamine were dissolved in 57.3 ml DMF i.e. in the remaining amount of DMF. The stirring rate of the solved pre-polymer was increased and then the solution of amine/DMF was added at once, and a substantial increase in viscosity was noted.

After de-gassing the viscosity was measured to about 19340 mPas. A spinning test was carried out, and the resulting fibre was hard to stretch. Specific strength: about 15.4 cN/Tex.

To the remaining solution of polymer trifluoro acetic acid anhydride (TFA-anhydride) was added to prevent gelling. About three days later a spinning test was also made with this polymer solution, this time it was easier to stretch the fibre. Specific strength: about 16.2 cN/Tex.

The prepared polymer "POL 4040" has 0.88 mmol secondary OH-groups per gram polymer.

The molecular weight of the polymer was estimated by SEC (Size Exclusion Chromatography) in DMF-LiCl against PEO-standards and was found to be 68500.

### Example 2

### Coupling of benzyl penicillin to the product from example 1, polymer "POL 4040" in solution.

14.3 g of the product from example 1, polymer "POL 4040", (corresponding to 2 mmol OH-groups) was mixed with 0.74 g (2 mmol) benzyl penicillin. Then 0.42 g (2.2 mmol) of a water soluble carbodiimide was added and a catalytic amount of dimethylaminopyridine. The reaction mixture was stirred during three days, then was 50 ml ethanol added. At the addition of ethanol the polymer with covalently bonded benzyl penicillin immediately precipitated. The precipitated polymer was washed with ethanol, distilled water and ethanol again. The precipitated polymer was dissolved in 30 ml dimethylformamide and was precipitated again with ethanol. The again precipitated polymer was dissolved in 28 ml dimethylformamide, then a film was prepared of the whole solution. The film was dried in vacuum on a glass surface and was put in a water bath to be released from the glass surface and was washed once more. SEC (Size Exclusion Chromatography) did not show any obvious difference in relation to the molecular weight.

Analysis of sulphur gave that 0.027 mmol OH-groups per gram polymer was bonded to benzyl penicillin, which corresponds to 9 mg benzyl penicillin per gram polymer. Thus, 2.5 % of the OH-groups was bonded to benzyl penicillin.

### Example 3

### Coupling of benzyl penicillin to the product from example 1, polymer "POL 4040" in the form of an open porous polymer material, a so called foam.

An open porous polymer material of the product from example 1 was prepared according to Swedish Patent Application SE, A, 0004856-1, which is hereby referred to as a whole. SE, A, 0004856-1 describes among other methods for preparing an open porous polymer material. To 0.9 g of the product from example 1 in the form of an open porous polymer material (a so-called foam, here OH-polymer foam) 0.73 g (ca 2 mmol) benzyl penicillin, 0.42 g (2.2 mmol) EDC-HCl (water soluble carbodiimide), a catalytic amount of dimethylaminopyridine and 10 ml of distilled water was added. All that was added went to solution and was soaked up by the open porous polymer material (OH-polymer foam). The reaction was protected from light and was allowed to continue for three days, then the open porous polymer material was washed with water and ethanol several times before the polymer material was dried in vacuum.

Analysis of sulphur gave that 0.038 mmol OH-groups per gram polymer, in the form of an open porous polymer material, was bonded to benzyl penicillin, which corresponds to 12.4 mg benzyl penicillin per gram polymer.

A further experiment was done, wherein about 1 g of the product from example 1 in the form of an open porous polymer material was used, principally this experiment was identical to the experiment immediately above, i.e. to about 1 g of the product from example 1 in the form of an open porous polymer material (a so-called foam, here an OH-polymer foam) 0.73 g (about 2 mmol) benzyl penicillin, 0.42 g (2.2 mmol) EDC-HCl (water soluble carbodiimide), a catalytic amount of dimethylaminopyridine and 10 ml of distilled water was added. All that was added went to solution and the solution was soaked up by the open porous polymer material (OH-polymer foam). The reaction was protected from light and was allowed to go on for three days, then the open porous polymer material was carefully washed with water and ethanol several times before the polymer material was dried.

Sulphur analysis gave that the polymer material contained 0.146 % by weight of sulphur, which corresponds to 1.46 % by weight of benzyl penicillin, and this is equivalent with that 5 % of the OH-groups of the polymer material has bonded benzyl penicillin.

The polymer material from this further experiment was compared with an untreated polymer material, i.e. without bezyl penicillin, in a test in vitro with benzyl penicillin sensitive bacteria (Micrococcus luteus ATCC 9341). The polymer material from this further experiment did show a clear zone without growth of bacteria and the untreated polymer material did not show any effects of the bacteria.

### Example 4

### Coupling of glycine to the product from example 1, i.e. a polymer prepared from a pre-polymer (2 MDI + PCL 530) chain extended with 1,3-diamino-2-hydroxypropane in dimethylformamide.

To 71.5 g of the titled polymer solution, i.e. the product from example 1, which corresponds to 10 mmol OH-groups, 4.18 g (20 mmol) of benzyloxycarbonylglycine and 8.9 g (20 mmol) of the coupling reactant benzotriazole-1-yl-oxy-tris-(dimethylamino)-phosphoniumhexafluorophosphat (BOP) were added, then 3.1 g (30 mmol) of triethylamine was added. The mixture was stirred for two days, then it was precipitated in 220 ml abs ethanol. The precipitated polymer was washed with 3x50 ml ethanol and was held in ethanol for three days before it was dried and solved in 30 ml dry DMF.

To the new polymer solution 1.6 g ammoniumformiate, 25 mmol, was added and then 100 mg palladium on active carbon, Pd/C 10 %. To decrease the viscosity 10 ml DMF and 30 ml ethanol were added. The mixture was heated at 45-50°C for 5-6 hours.

It was possible to observe gas development visually. The catalyst (Pd/C) was filtered away and the remaining polymer solution was precipitated with 100 ml ethanol. The precipitated polymer was washed with ethanol and water, dried and solved in 100 ml DMF. After a further precipitation in ethanol, washing with water, ethanol and solving in DMF, the polymer was precipitated in water, washed in ethanol, dried and solved in 30 ml dried DMF. From this polymer solution a film was made which was dried in a vacuum oven at 50°C. Samples of this film was sent for amino acid analysis. The film contained 0.44 mmol glycin per gram polymer (theoretically 0.88 mmol OH-groups/gram polymer), i.e. 50 % of OH-groups did bond glycin.

### Example 5

### Coupling of 6-aminohexanacid to the product from example 1, a polymer prepared from a pre-polymer (2 MDI + PCL 530) chain extended with 1,3-diamino-2-hydroxypropane in dimethylformamide.

To 29 g of the titled polymer solution, i.e. the product from example 1 which corresponds to 4.05 mmol OH-groups, 2.12 g (8 mmol) benzyloxycarbonyl protected 6-aminohexan acid (Z-6-Aca-OH), 3.53 g (8 mmol) of the coupling reactant BOP were added, and 1.1 g (about 11 mmol) trietylamin was added.

The mixture was stirred for more than 48 hours, then it was precipitated in ethanol and washed with 3x30 ml ethanol. The precipitated polymer was dissolved in 30 ml dry DMF. To the new polymer solution in DMF 1g (15 mmol) ammoniumformiat and 50 mg Pd/C, 10 %, and 10 ml ethanol were added. The mixture was heated at about 50°C for three days under stirring.

The reaction was processed in analogy with example 4, but using half of the amounts. Finally a film was prepared and vacuum-dried at 50°C before it was sent to aminoacid analysis. The result was that 0.1 mmol aminohexan acid per gram polymer were bonded (theoretically 0.8 mmol OH-groups/g), i.e. 11 % of the OH-groups.

### Example 6

### Coupling of GHK, glycylhistidyllysin, to the product from example 1, i.e. a polymer prepared from a pre-polymer (2 MDI + PCL 530) chain extended with 1,3-diamino-2-hydroxypropane in dimethylformamide.

To 35.7 g of the titled polymer solution, i.e. the product from example 1, which corresponds to 5 mmol OH-groups/gram polymer solution, 0.321 g (0.5 mmol) tert-butyloxycarbonyl-protected GHK and 225 mg (0.5 mmol) of the coupling reactant BOP. Then an equivalent amount of triethylamine was added, i.e. 50 mg. The mixture was stirred for 72 hours at room temperature, then it was treated with 5 ml triofluoracetic acid, TFA. This mixture was stirred to the next day and then precipitated in 60 ml absolute ethanol. The precipitated polymer was washed with ethanol, water, dried and solved in dry DMF. This treatment was repeated once more, then was a film prepared, and dried in a vacuum oven and sent for amino acid analysis. The result: 0.037 mmol tripeptid/g polymer film. The polymer solution contained 5.64 g polymer. Exchange of coupling: about 40 %.

### Example 7

### Coupling of heparin to the product from example 1, polymer "POL 4040" in the form of an open porous polymer material, a so called foam.

For the preparation of an open porous polymer material of the product from example 1 it is here referred like before in example 3, to the Swedish Patent Application SE, A, 0004856-1.

To approx. 0.6 g of the open porous polymer material (polymer foam) prepared as above, based on the chain extender 1,3-diamino-2-hydroxypropane (OH-handle: 0.88 mmol secondary OH-groups per gram polymer), a solution of 0.25 g of heparin (Mw-5000), 10 mg of EDC-HCl (water soluble carbodiimide) and 5 mg of 4-dimethylaminopyridine (DMAP) in 10 ml of distilled water was added. The solution was soaked up by the open porous polymer material (the polymer foam) and was left for 72 hours. The open porous polymer material was then washed carefully several times with water and ethanol prior to drying. Sulphur analysis gave a sulphur content of 0.155 % by weight, which corresponds to 1.35 % by weight of heparin.

### Example 8

### Coupling of biotin to the product from example 1, polymer "POL 4040" in the form of an open porous polymer material, a so called foam.

For the preparation of an open porous polymer material of the product from example 1 it is hereby referred to, like earlier in example 3, to the Swedish Patent Application SE, A, 004856-1.

To approx. 0.5 g of polymer foam prepared as above, based pn the chain extender 1,3-diamino-2-hydroxypropane (OH-handle, 0.99 mmol secondary OH-groups per gram polymer), a solution of 0.24 g of biotin (1 mmol), 0.23 g of EDC-HCl (1.2 mmol)and 0.26 g of N,N-diisopropylethylamine (2 mmol) in 5 ml of distilled water were added. The solution was soaked up by the foam and was left for 24 hours. The foam (the open porous polymer material) was then washed carefully several times with water and ethanol was then allowed to dry. Sulphur analysis: Sulphur content is 0.06 % by weight which corresponds to 0.45 % biotin by weight. The presence of biotin was further established by forming the complex with the protein avidin followed by amino acid analysis.

### Example 9

### An in vitro concept test to decide anti-bacterial effect at polymer films with coupled benzyl penicillin through a pilot test of polymer films with benzyl penicillin (benzyl PC) and Gentamicin respectively.

A polymer film with coupled benzyl penicillin (PC G) was prepared in a corresponding way to example 2, wherein the polymer film contains 7 mg benzyl penicillin/g polymer film.

A polymer film with water soluble Gentamicin was prepared, wherein in the polymer film contains 0.12 g Gentamicin/g polymer film.

Polymer films without antibiotic were prepared as control.

The polymer films were placed on a substrate with bacteria which were sensitive to the respective antibiotic. Two plates were used for each film, a blood plate and a plasma plate.

Two different bacteria species were used: Micrococcus luteus ATCC 9341 and Coagulas negative staphylococcs g+(plate marked CK) CCUG (controlled culture of Gothenburg) 6388.

After incubation for 24 hours all plates were photographed. No effect on the bacteria was observed for the plates used for control (only polymer film) but all tests, i.e. polymer films with benzyl penicillin (Benzyl PC) and Gentamicin respectively, as above, showed a cleared zone (to a different extent) without bacterial growth.

## Claims

1. A linear block polymer having a molecular weight of at least 10⁴ Dalton, which linear block polymer is comprised of internally linearly connected sequences, which sequences may be described according to formula (1) 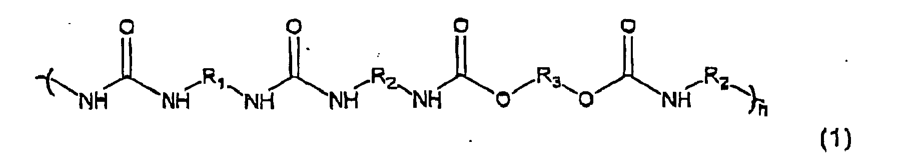 wherein
1 to 100 % of R₁, which 1 to 100 % of R₁ may be the same or different, comprise a segment which is described according to formula (1a) 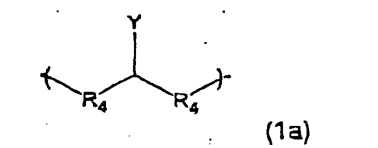 wherein Y, which is a substituent, is R₅-Z, wherein
R₅ is (C₀-C₈) alkyl, and
each Z, which Z may be the same or different, is a protected or unprotected functional group such as hydroxyl, amino, carboxyl, thiol, cyano, or nitro group, and
each R₄, which R₄ may be the same or different, is (C₁-C₆) alkyl; and,
when not 100 % of R₁ comprises a segment according to formula (1a) as above, may each other R₁, which other R₁, the same or different, do not comprise a segment according to formula (1a), be derived from aliphatic or aromatic diamines;
each R₂, which R₂ may be same or different, may be derived from a diisocyanate and comprises no, one or more ester groups;
each R₃, which R₃ may be same or different, comprises no, one or more ester groups and may be derived from one or more of polyesterdiol, polyetherdiol or monodiol, or
each R₃, which R₃ may be the same or different, may be described according to formula (1b) 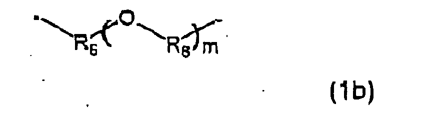 wherein each R₅ is (C₂-C₄) alkyl, and
m is 1 to 6;
wherein each R₂ and/or each R₃ comprise one or more ester groups; and
n is in the interval from 10 to 1000 which gives said linear block polymer a molecular weight of at least 10⁴ Dalton.

2. A linear block polymer according to claim 1, wherein R₅ is missing, or alternatively may be said to be a bond, i.e., R₅ is C₀-alkyl.

3. A linear block polymer according to claim 1 or 2, wherein 10 to 100 % of R₁, which 10 to 100 % of R₁ may be the same or different, comprises a segment which may be described according to formula (1a).

4. A linear block polymer according to any one of preceding claims, wherein each R₁ which R₁ may be the same or different, comprises a segment which may be described according to formula (1a).

5. A linear block polymer according to any one of preceding claims, wherein each R₂, which R₂ may be the same or different, may be derived from diphenylmethanedilsocyanat (MDI), hexamethylenediisocyanat (HDI), 1-isocyanato-4-[(4-isocyanatocyclohexyl)methyl]cyclohexane (H₁₂MDI) or ethyl-2,6-diisocyanatohexanoate (LDI).

6. A linear block polymer according to any one of preceding claims, wherein R₃ may be derived from one or more of polytetramethyleneoxidadiol, polyethyleneoxidedlol, polycaprolactonediol, polyethyleneglycoladipatediol, polyldiethyleneglycoladipatdiol, glycerinemonoallylether, trimethylolpropanemonallylether, glycerinemonoglycidylether, dimethylolpropionacid methylester, dimethylolpropionacidbrombutylester, esters of monocarboxymethylethers of glycerine, or trimethylpropane.

7. A linear block polymer according to any one of preceding claims, wherein each Z, which Z may be the same or different, is a hydroxyl, amino, carboxyl, thiol, cyano, or nitro group.

8. A linear block polymer according to any one of preceding claims, wherein said linear block polymer has a molecular weight of at least 5*10⁴ Dalton.

9. A linear block polymer according to any one of preceding claims, wherein said linear block polymer comprises a biologically active substance, wherein said biologically active substance Is covalently bonded to Z.

10. A linear block polymer according to any one of preceding claims, wherein said biologically active substance for example may be biologically active peptides e.g. growth factors or GHK, i,e. glycylhistidyllysin, penicillins e.g. benzylpenicillin, fucidin acid or tetracyclins.

11. A method for preparation of a linear block polymer according to any one of claim 1 to 10, wherein said method comprises chain-extension, wherein about n mol of a diamine according to formula (2) 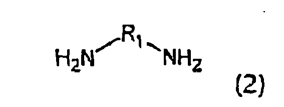 is reacted with about n mol of a urethanediisocyanate according to formula (3), 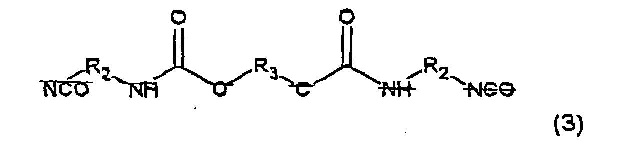 wherein R₁, R₂ and R₃ are defined as in formula (1), provided that if R₁ comprises R₅₋Z which may interfere with the preparation then R₅-Z is protected.

12. A method for preparing according to claim 11 or a method for preparing a linear block polymer according to claim 1, wherein said method comprises precipitation with alcohol, such as ethanol or isopropanol, or precipitation with water.

13. A method for preparing according to claim 12, wherein said method further comprises covalent bonding of a biologically active substance to said linear block polymer, for example through primary or secondary functional groups for covalent bonding.

14. A method for preparing according to claim 13, wherein said covalent bonding comprises bonding to functional groups for covalent bonding at said linear block polymer.

15. A method for fractional precipitation of a linear block polymer according to any one of claim 1 to 10, wherein the method comprises precipitation and solving of said linear block polymer and thus the molecular weight distribution of said linear block polymer may be changed.

16. Use of a linear block polymer according to any one of claim, to 10, wherein said use is use for preparation of, for example, fibres, film, moulded bodies, implants, pipes or an open porous polymer material, materials in implants for humans or animals, for example, implants for tendons, ligaments, blood vessels, discs or menisci.

17. Use of a linear block polymer according to any one of claim 1 to 10, in pharmaceutical compositions, at microencapsulation, in suspensions, in emulsions, in porous polymer materials or the like, or wherein said use is use for preparation of:
• filling material for example in bone such as discs, synthetic bone replacement, for example in the form of granules for replacement of bone tissue, menisci or the like,
• pipes for, for example, replacement of blood vessels, guidance for promotion/regeneration of tendons and/or nerves, or other biological tissue,
• in connection to treatment of wounds, carrier of dressings for wound healing, growth factors or the like, artificial skin, or
• matrix/scaffold for, for example; stem cells, fibroblasts, osteablasts, osteocytes, condroblasts, condrocytes among other, as well as autogenous, allogenous as xenogenous, or matrix/scaffold for promotion/regeneration of tissue, for example, tendons and/or nerves, or other biological tissue.

18. Use of a linear block polymer according to any one of claim 1 to 10, wherein said use is use for preparation of a material for promoting the healing of wounds in humans or animals.

19. Implants for humans or animals, which implants comprises a linear block polymer according to any one of claim 1 to 10.

20. Material for filling in banes, promotion of wound healing, replacement for blood vessels, guidance for growth/regeneration of tendons and/or nerves, or other biological tissue in humans or animals, which material comprises a linear block polymer according to any one of claim 1 to 10.

21. Pharmaceutical compositions, microencapsules, suspensions, emulsions, matrix/scaffolds for growth/regeneration of tissue, for example, tendons and/or nerves, or other biological tissue, which comprises a linear block polymer according to any one of claim 1 to 10.

22. A porous polymer material, which polymer material comprises a linear block polymer according to any one of claim 1 to 10.

## Patentansprüche

1. Lineares Blockpolymer mit einem Molekulargewicht von mindestens 10⁴ Dalton, welches lineare Blockpolymer aus intern linear verbundenen Sequenzen gebildet ist, welche Sequenzen mit der Formel (1) beschrieben werden können 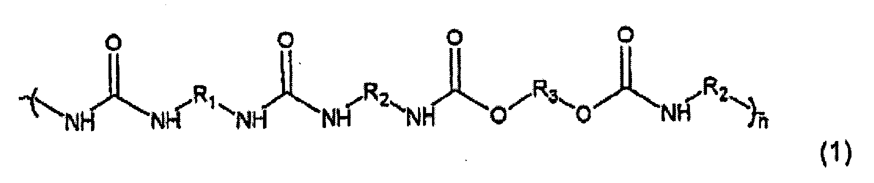 worin
1 bis 100 % R₁, welche 1 bis 100 % R₁ gleich oder verschieden sein können, ein Segment umfassen, das mit der Formel (1 a) beschrieben wird 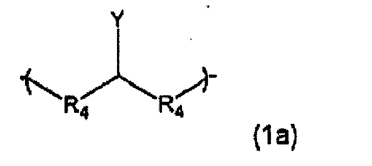 worin Y, das ein Substituent ist, R₅-Z ist, worin R₅ (C₀-C₆) Alkyl ist und
jedes Z, welches Z gleich oder verschieden sein kann, eine geschützte oder ungeschützte funktionelle Gruppe ist wie Hydroxyl-, Amino-, Carboxyl-, Thiol-, Cyano- oder Nitrogruppe und
jedes R₄, welches R₄ gleich oder verschieden sein kann, (C₁-C₆) Alkyl ist, und
wenn nicht 100 % R₁ ein Segment nach Formel (1 a) wie oben umfassen, kann jedes andere R₁, welches andere R₁, gleich oder verschieden, kein Segment nach Formel (1 a) umfasst, von aliphatischen oder aromatischen Diaminen abgeleitet sein;
jedes R₂, welches R₂ gleich oder verschieden sein kann, kann von einem Diisocyanat abgeleitet sein und umfasst keine, eine oder mehrere Estergruppe(n);
jedes R₃, welches R₃ gleich oder verschieden sein kann, umfasst keine, eine oder mehrere Estergruppe(n) und kann von einem oder mehreren von Polyesterdiol, Polyetherdiol oder Monodiol abgeleitet sein, oder
jedes R₃, welches R₃ gleich oder verschieden sein kann, kann mit Formel (1 b) beschrieben werden 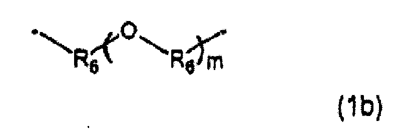 worin jedes R₄ (C₂-C₄) Alkyl ist und
m 1 bis 6 ist;
worin jedes R₂ und/oder jedes R₃ eine oder mehrere Estergruppen umfasst; und
n im Intervall von 10 bis 1000 liegt, was dem linearen Blockpolymer ein Molekulargewicht von mindestens 10⁴ Dalton gibt.

2. Lineares Blockpolymer nach Anspruch 1, worin R₅ fehlt, oder alternativ als eine Bindung bezeichnet werden kann, d. h. R₅ ist C₀-Alkyl.

3. Lineares Blockpolymer nach Anspruch 1 oder 2, worin 10 bis 100 % R₁, welche 10 bis 100 % R₁ gleich oder verschieden sein können, ein Segment umfassen, das mit der Formel (1 a) beschrieben werden kann.

4. Lineares Blockpolymer nach einem der vorhergehenden Ansprüche, worin jedes R₁, welche R₁ gleich oder verschieden sein können, ein Segment umfasst, das mit der Formel (1 a) beschrieben werden kann.

5. Lineares Blockpolymer nach einem der vorhergehenden Ansprüche, worin jedes R₂, welche R₂ gleich oder verschieden sein können, von Diphenylmethandiisocyanat (MDI), Hexamethylendiisocyanat (HDI), 1-Isocyanato-4-[(4-isocyanatocyclohexyl)methyl]cyclohexan (H₁₂MDI) oder Ethyl-2,6-diisocyanatohexanoat (LDI) abgeleitet sein kann.

6. Lineares Blockpolymer nach einem der vorhergehenden Ansprüche, worin R₃ von einem oder mehreren von Polytetramethylenoxiddiol, Polyethylenoxiddiol, Polycaprolactondiol, Polyethylenglycoladipatdiol, Polydiethylengylcoladipatdiol, Glycerinmonoallylether, Trimethylolpropanmonoallylether, Glycerinmonoglycidylether, Dimethylolpropionsäuremethylester, Dimethylolpropionsäurebrombutylester, Estern von Monocarboxymethylethern von Glycerin oder Trimethylpropan abgeleitet sein können.

7. Lineares Blockpolymer nach einem der vorhergehenden Ansprüche, worin jedes Z, welche Z gleich oder verschieden sein können, eine Hydroxyl-, Amino-, Carboxyl-, Thiol-, Cyano- oder Nitrogruppe ist.

8. Lineares Blockpolymer nach einem der vorhergehenden Ansprüche, worin das lineare Blockpolymer ein Molekulargewicht von mindestens 5*10⁴ Dalton aufweist.

9. Lineares Blockpolymer nach einem der vorhergehenden Ansprüche, worin das lineare Blockpolymer eine biologisch aktive Substanz umfasst, worin die biologisch aktive Substanz kovalent an Z gebunden ist.

10. Lineares Blockpolymer nach einem der vorhergehenden Ansprüche, worin die biologisch aktive Substanz zum Beispiel biologisch aktive Peptide sein können, z. B. Wachstumsfaktoren oder GHK, d. h. Glycylhistidyllysin, Penicilline, z. B. Benzylpenicillin, Fucidinsäure oder Tetracycline.

11. Verfahren zur Herstellung eines linearen Blockpolymers nach einem der Ansprüche 1 bis 10, worin das Verfahren Kettenverlängerung umfasst, worin ungefähr n Mol eines Diamins nach Formel (2) 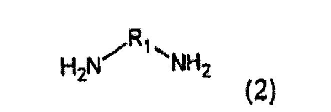 mit ungefähr n Mol eines Urethandiisocyanats nach Formel (3) umgesetzt werden 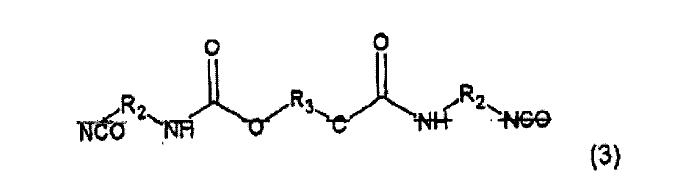 worin R₁, R₂ und R₃ wie in Formel (1) definiert sind, vorausgesetzt, dass wenn R₁ R₅-Z umfasst, das bei der Herstellung stören kann, dann R₅-Z geschützt ist.

12. Verfahren zur Herstellung nach Anspruch 11 oder ein Verfahren zur Herstellung eines linearen Blockpolymers nach Anspruch 1, worin das Verfahren Ausfällung mit Alkohol, wie Ethanol oder Isopropanol oder Ausfällung mit Wasser umfasst.

13. Verfahren zur Herstellung nach Anspruch 12, worin das Verfahren ferner kovalente Bindung einer biologisch aktiven Substanz an das lineare Blockpolymer umfasst, zum Beispiel durch primäre oder sekundäre funktionelle Gruppen zur kovalenten Bindung.

14. Verfahren zur Herstellung nach Anspruch 13, worin die kovalente Bindung Bindung von funktionellen Gruppen zur kovalenten Bindung an das lineare Blockpolymer umfasst.

15. Verfahren zur fraktionierten Fällung eines linearen Blockpolymers nach einem der Ansprüche 1 bis 10, worin das Verfahren Ausfällung und Lösen des linearen Blockpolymers umfasst und auf diese Weise die Molekulargewichtsverteilung des linearen Blockpolymers verändert werden kann.

16. Verwendung eines linearen Blockpolymers nach einem der Ansprüche 1 bis 10, worin die Verwendung Verwendung zur Herstellung von, zum Beispiel, Fasern, Filmen, Formkörpern, Implantaten, Röhren oder einem offenporigen Polymermaterial, Materialien in Implantaten für Menschen oder Tiere, zum Beispiel, Implantate für Sehnen, Bänder, Blutgefäße, Scheiben oder Menisken ist.

17. Verwendung eines linearen Blockpolymers nach einem der Ansprüche 1 bis 10, in pharmazeutischen Zusammensetzungen, bei Mikroverkapselung, in Suspensionen, in Emulsionen, in porösen Polymermaterialien oder dergleichen, oder worin die Verwendung Verwendung bei der Herstellung von:
• Füllmaterial zum Beispiel in Knochen wie Scheiben, synthetischer Knochenersatz, zum Beispiel in Form von Granulat zum Ersatz von Knochengewebe, Menisken oder dergleichen,
• Röhren zum Beispiel zum Ersatz von Blutgefäßen, Führung zur Förderung/Regeneration von Sehnen und/oder Nerven oder anderem biologischen Gewebe,
• Trägern von Abdeckungen zur Wundheilung, Wachstumsfaktoren oder dergleichen, künstlicher Haut in Verbindung mit Behandlung von Wunden, oder
• Matrix/Gerüst zum Beispiel für Stammzellen, Fibroblasten, Osteoblasten, Osteocyten, Condroblasten, Condrocyten unter anderen, sowie Autogene, Allogene wie Xenogene oder Matrix/Gerüst zur Förderung/Regeneration von Gewebe, zum Beispiel Sehnen und/oder Nerven oder anderem biologischen Gewebe ist.

18. Verwendung eines linearen Blockpolymers nach einem der Ansprüche 1 bis 10, worin die Verwendung Verwendung zur Herstellung eines Materials zur Förderung der Wundheilung bei Menschen oder Tieren ist.

19. Implantate für Menschen oder Tiere, welche Implantate ein lineares Blockpolymer nach einem der Ansprüche 1 bis 10 umfassen.

20. Material zur Füllung von Knochen, Förderung von Wundheilung, Ersatz für Blutgefäße, Führung für Wachstum/Regeneration von Sehnen und/oder Nerven, oder anderem biologischen Gewebe bei Menschen oder Tieren, welches Material ein lineares Blockpolymer nach einem der Ansprüche 1 bis 10 umfasst.

21. Pharmazeutische Zusammensetzungen, Mikrokapseln, Suspensionen, Emulsionen, Matrix/Gerüste für Wachstum/Regeneration von Gewebe, zum Beispiel Sehnen und/oder Nerven, oder anderem biologischen Gewebe, welche ein lineares Blockpolymer nach einem der Ansprüche 1 bis 10 umfassen.

22. Poröses Polymermaterial, welches Polymermaterial ein lineares Blockpolymer nach einem der Ansprüche 1 bis 10 umfasst.

## Revendications

1. Polymère séquencé linéaire ayant une masse moléculaire d'au moins 10⁴ D, lequel polymère séquencé linéaire est constitué de séquences reliées linéairement de façon interne, dont les séquences peuvent être décrites selon la formule (1) 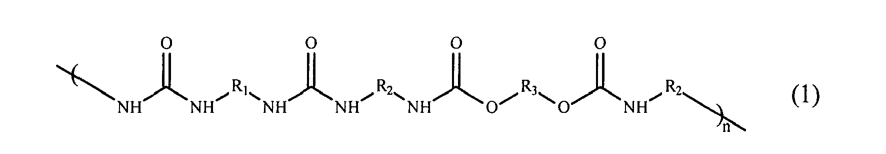 dans laquelle
1 à 100 % de R₁, dont 1 à 100 % de R₁ peut être le même ou différent, constituent un segment qui est décrit selon la formule (1a) 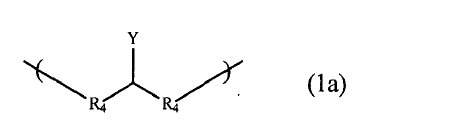 dans laquelle Y, qui est un substituant, est R₅-Z, où
R₅ est un alkyle en C₀-C₈, et
chaque Z, qui peut être un Z identique ou différent, est un groupe fonctionnel protégé ou non protégé tel qu'un groupe hydroxyle, amino, carboxyle, thiol, cyano ou nitro, et
chaque R₄, qui peut être un R₄ identique ou différent, est un alkyle en C₁₋C₆ ; et,
quand pas 100 % de R₁ constitue un segment selon la formule (1a) ci-dessus, chaque autre R₁, qui est un autre R₁, identique ou différent qui ne contient pas un segment selon la formule (1a) peut être issu de diamines aliphatiques ou aromatiques ; chaque R₂, dont R₂ peut être identique ou différent, peut être dérivé d'un diisocyanate et comprendre aucun, un ou plusieurs groupes ester ;
chaque R₃, dont R₃ peut être identique ou différent, comprend aucun, un ou plusieurs groupes ester et peut être dérivé d'un ou plusieurs polyesterdiol, polyétherdiol ou monodiol, et
chaque R₃, dont R₃ peut être identique ou différent, peut être représenté selon la formule (1b) 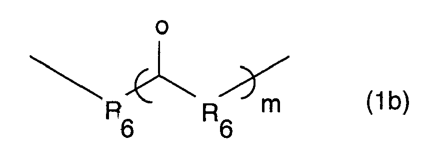 dans laquelle, R₆ est un alkyle en C₂-C₄, et
m est 1 à 6 ;
**caractérisé en ce que** R₂ et/ou R₃ comprennent un ou plusieurs groupes ester ; et n est dans l'intervalle 10 à 1000 qui donne audit polymère séquencé une masse moléculaire d'au moins 10⁴ daltons.

2. Polymère séquencé linéaire selon la revendication 1, **caractérisé en ce que** R₅ est manquant, ou autrement peut être dit être une liaison, c.a.d., R₅ est un alkyle en C₀.

3. Polymère séquencé linéaire selon la revendication 1 ou 2, **caractérisé en ce que** 10 à 100 % de R₁, dont 10 à 100 % de R₁ peuvent être identiques ou différents, comprend un segment qui peut être décrit selon la formule (1a).

4. Polymère séquencé linéaire selon l'une quelconque des revendications précédentes, **caractérisé en ce que** chaque R₁, dont R₁ peut être identique ou différent, comprend un segment qui peut être décrit selon la formule (1a).

5. Polymère séquencé linéaire selon l'une quelconque des revendications précédentes, **caractérisé en ce que** chaque R₂, dont R₂ peut être identique ou différent, peut être dérivé du diphénylméthane diisocyanate (MDI), de l'hexaméthylène diisocyanate (HDI), du 1-isocyanato-4-[(4-isocyanato cyclohexyl)méthyl]cyclohexane (H₁₂MDI) ou de l'éthyl-2,6-diisocyanato hexanoate (LDI).

6. Polymère séquencé linéaire selon l'une quelconque des revendications précédentes, **caractérisé en ce que** chaque R₃, dont R₃ peut être identique ou différent, peut être dérivé d'un ou plusieurs diol de poly(oxyde de tétraméthylène), diol de poly(oxyde d'éthylène), diol de polycaprolactone, diol de poly adipate d'éthylène glycol, diol de poly adipate de diéthylène glycol, monoallyl éther de glycérine, ester méthylique d'acide diméthylolpropionique, ester bromobutylique d'acide diméthylolpropionique, esters de monocarboxyméthyléthers de glycérine ou de triméthylpropane.

7. Polymère séquencé linéaire selon l'une quelconque des revendications précédentes, **caractérisé en ce que** chaque Z, dont Z peut être identique ou différent, est un groupe hydroxyle, amino, carboxyle, thiol, cyano ou nitro.

8. Polymère séquencé linéaire selon l'une quelconque des revendications précédentes, **caractérisé en ce que** le polymère séquencé linéaire a une masse moléculaire d'au moins 5 x 10⁻⁴ daltons.

9. Polymère séquencé linéaire selon l'une quelconque des revendications précédentes, **caractérisé en ce que** le polymère séquencé linéaire contient une substance active biologiquement, où laquelle substance active biologiquement est lié à Z de façon covalente.

10. Polymère séquencé linéaire selon l'une quelconque des revendications précédentes, **caractérisé en ce que** la substance active biologiquement peut être, par exemple, des peptides actifs biologiquement, par exemple, des facteurs de croissance ou GHK, c.a.d., de la glycylhistidyllysine, des pénicillines, par exemple, la benzyl pénicilline, l'acide fucidine ou les tétracyclines.

11. Procédé de préparation d'un polymère séquencé linéaire selon l'une quelconque des revendications 1 à 10, **caractérisé en ce que** ledit procédé comprend un allongement de chaîne dans lequel environ n moles d'une diamine selon la formule (2) 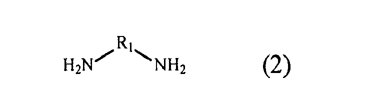 sont mises à réagir avec environ n moles d'un uréthane diisocyanate selon la formule (3), 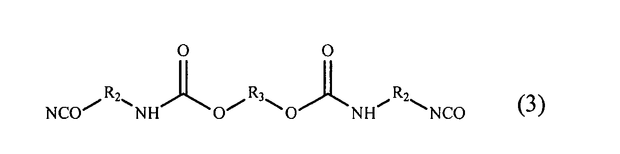 dans lesquelles R₁, R₂ et R₃ sont définis comme dans la formule (1), pourvu que si R₁ comprend R₅-Z qui peut interférer avec la préparation alors R₅-Z est protégé.

12. Procédé de préparation d'un polymère séquencé linéaire selon la revendication 11, ou procédé de préparation d'un polymère séquencé linéaire selon la revendication 1, **caractérisé en ce que** ledit procédé comprend une précipitation par un alcool, comme l'éthanol ou l'isopropanol, ou une précipitation par l'eau.

13. Procédé de préparation d'un polymère séquencé linéaire selon la revendication 12, **caractérisé en ce que** ledit procédé comprend en outre la liaison covalente d'une substance active biologiquement audit polymère séquencé linéaire, par exemples par des groupes fonctionnels primaires ou secondaires pour une liaison covalente.

14. Procédé de préparation d'un polymère séquencé linéaire selon la revendication 13, **caractérisé en ce que** ladite liaison covalente comprend la liaison de groupes fonctionnels pour une liaison covalente audit polymère séquencé linéaire

15. Procédé de précipitation fractionnée d'un polymère séquencé linéaire selon l'une quelconque des revendications 1 à 10, **caractérisé en ce que** le procédé comprend la précipitation et l'obtention dudit polymère séquencé linéaire et de la sorte la distribution en masses moléculaires dudit polymère séquencé linéaire peut être modifiée.

16. Utilisation d'un polymère séquencé linéaire selon l'une quelconque des revendications 1 à 10, **caractérisé en ce que** ladite utilisation est utilisée pour la préparation, par exemple, de fibres, d'un film, d'objets moulés, d'implants, de tuyaux ou d'un matériau polymère à porosité ouverte, des matériaux dans les implants pour humains ou animaux, par exemple, des implants pour tendons, ligaments, vaisseaux sanguins, disques ou ménisques.

17. Utilisation d'un polymère séquencé linéaire selon l'une quelconque des revendications 1 à 10, dans des compositions pharmaceutiques, pour microencapsulation, dans des suspensions, dans des émulsions, dans des matériaux polymère poreux ou autres, ou où ladite utilisation sert à la préparation de
• matériau de remplissage par exemple dans un os comme des disques, le remplacement par un os synthétique, par exemple sous forme de granulés pour remplacement d'un tissus osseux, de ménisques et autres,
• tuyaux, par exemple, pour le remplacement de vaisseaux sanguins, guidage pour la facilitation/régénération des tendons et/ou des nerfs ou d'un autre tissus biologique,
• en relation avec le traitement des blessures, support de pansement pour cicatrisation de blessure, facteurs de croissance ou autres, peau artificielle, ou
• une matrice/échafaudage, par exemple, pour cellules souches, fibroblastes, ostéoblastes, ostéocytes, condroblastes, condrocytes parmi d'autres, aussi bien qu'autogène, allogène que xénogène, ou matrice/ échafaudage pour la facilitation/régénération de tissus, par exemple, des tendons et/ou des nerfs, ou un autre tissus biologiques.

18. Utilisation d'un polymère séquencé linéaire selon l'une quelconque des revendications 1 à 10, **caractérisée en ce que** ladite utilisation est une utilisation pour la préparation d'un matériau pour faciliter la cicatrisation de blessures chez les humains ou les animaux.

19. Implants pour les humains ou les animaux, lesquels implants contiennent un polymère séquencé linéaire selon l'une quelconque des revendications 1 à 10.

20. Matériau pour le remplissage des os, la facilitation de la cicatrisation d'une blessure, le remplacement de vaisseaux sanguins, le guidage pour la croissance/régénération de tendons et/ou de nerfs, ou autre tissus biologique chez les humains ou les animaux, lequel matériau contient un polymère séquencé linéaire selon l'une quelconque des revendications 1 à 10.

21. Compositions pharmaceutiques, microcapsules, suspensions, émulsions, matrices/échafaudage pour croissance/régénération, par exemple de tendons et/ou de nerfs, ou autre tissus biologique; qui contiennent un polymère séquencé linéaire selon l'une quelconque des revendications 1 à 10.

22. Matériau polymère poreux, lequel polymère contient un polymère séquencé linéaire selon l'une quelconque des revendications 1 à 10.
